# EUROPÄISCHE PATENTSCHRIFT

(11) **EP 1 332 769 B1**
(45) Veröffentlichungstag und Bekanntmachung des Hinweises auf die Patenterteilung: **16.01.2008**
(21) Anmeldenummer: 03000486.5
(22) Anmeldetag: 11.01.2003
(51) Int. Cl.: A61M 16/00

(54) **Verfahren und Vorrichtung zur Bereitstellung von Atemgas**
Method and apparatus for breathing gas preparation
Methode et appareil de préparation de gaz respiratoire

(30) Priorität: 01.02.2002 DE 10204098
(43) Veröffentlichungstag der Anmeldung: 06.08.2003
(73) Patentinhaber: Weinmann Geräte für Medizin GmbH & Co. KG, 22525 Hamburg (DE)
(72) Erfinder: Wedler, Wolfgang, 21147 Hamburg (DE)
(74) Vertreter: Klickow, Hans-Henning

(56) Entgegenhaltungen:
- GB-A- 1 010 420
- US-A- 4 176 663

## Beschreibung

Die Erfindung betrifft ein Verfahren zu Bereitstellung von Atemgas, bei dem das Atemgas aus einer Umgebung angesaugt und von mindestens zwei Hubförderern in Richtung auf eine Beatmungsmaske abgegeben wird, sowie bei dem die Hubförderer hinsichtlich ihrer Bewegungsabläufe relativ zueinander zeitlich phasenversetzt angesteuert werden und bei dem von einem zweiten Hubförderer ein Druck vor einer Rücklaufbewegung eines ersten Hubförderers erzeugt wird.

Die Erfindung betrifft darüber hinaus eine Vorrichtung zur Bereitstellung von Atemgas, die mindestens zwei Hubförderer aufweist, die über mindestens eine Atemgasleitung an eine Beatmungsmaske anschließbar sind, und bei der ein erster Hubförderer mit einem ersten Antrieb und ein zweiter Hubförderer mit einem zweiten Antrieb gekoppelt ist sowie bei der beide Antriebe an eine Bewegungskoordinierung angeschlossen sind sowie von den Hubförderern zu durchlaufende Bewegungszyklen relativ zueinander einen zeitlichen Phasenversatz aufweisen

Zur Bereitstellung von Atemgas für Beatmungsgeräte werden unterschiedliche Verfahren und Vorrichtungen angewendet. Typischerweise kann die Bereitstellung von Atemgas unter Verwendung von Gebläsen, Bälgen oder Kolben-Zylinder-Anordnungen erfolgen. Die Verwendung von Gebläsen weist den Vorteil von vergleichsweise niedrigen Preisen sowie einer sehr einfachen Konstruktion und Ansteuerung bei gleichzeitig hoher Zuverlässigkeit auf. Die Verwendung von Bälgen oder Kolben-Zylinder-Anordnungen besitzt den Vorteil, daß eine exakte Volumenkontrolle möglich ist, nur geringe Geräusche erzeugt werden und daß ein geringer Energiebedarf vorliegt. Nachteilig bei den bekannten Bälgen und Kolben-Zylinder-Anordnungen ist es, daß aufgrund der durchgeführten Hubbewegungen kein zeitlich konstanter Druck sowie Volumenfluß bereitgestellt werden kann und daß nur ein relativ begrenztes Hubvolumen zur Verfügung steht und nur eingeschränkt eine Leckagekompensation realisierbar ist.

Aus der US-A 4,176,663 sind ein Verfahren und eine Vorrichtung zur Bereitstellung von Atemgas unter Verwendung von zwei Hubförderern bekannt. Die Hubförderer sind als Bälge ausgebildet und jeweils mit einem Antrieb versehen. Eine Kopplung der Hubförderer erfolgt unter Verwendung von Ventilen, um zu verhindern, daß bei einer zeitlich versetzten Ansteuerung Gas von einem Hubförderer zum anderen strömt.

Aus der GB-A-1 010 420 ist bereits ein Verfahren zur Bereitstellung von Atemgas bekannt, bei dem das Atemgas aus einer Umgebung angesaugt und von mindestens zwei Hubförderern abgegeben wird. Die Hubförderer werden hinsichtlich ihrer Bewegungsabläufe relativ zueinander zeitlich phasenversetzt angesteuert. Von einem zweiten Hubförderer wird ein Druck vor einer Rücklaufbewegung eines ersten Hubförderers erzeugt.

Aufgabe der vorliegenden Erfindung ist es, ein Verfahren der einleitend genannten Art derart zu verbessern, daß sowohl eine exakte Volumenkontrolle als auch ein zeitlich konstanter Druck- sowie Volumenflußverlauf ermöglicht wird.

Diese Aufgabe wird erfindungsgemäß dadurch gelöst, die Hubförderer von einer gemeinsamen Regelung positioniert werden und daß der erste Hubförderer mit einem ersten Antrieb und der zweiten Hubförderer mit einem zweiten Antrieb gekoppelt wird.

Weitere Aufgabe der vorliegenden Erfindung ist es, eine Vorrichtung der einleitend genannten Art derart zu konstruieren, daß verbesserte Betriebseigenschaften bereitgestellt werden.

Diese Aufgabe wird erfindungsgemäß dadurch gelöst, daß die Bewegungskoordinierung als Regelung ausgebildet ist.

Durch die Verwendung von mindestens zwei relativ zueinander phasenversetzt angesteuerten Hubförderern ist es möglich, sowohl eine genaue Volumenkontrolle durchzuführen als auch einen leisen Betrieb und einen geringen Energiebedarf zu realisieren. Der geringe Energiebedarf ermöglicht insbesondere bei einem mobilen Betrieb eine Versorgung unter Verwendung von Akkumulatoren. Es kann eine Druck- und / oder eine Fluß-Kurve bereitgestellt werden.

Leckagen können auch bei Verwendung von kleinen Hubförderern beliebig ausgeglichen werden. Der Einsatz von Ausatemsystemen (künstliche Leckage im Schlauch-/ Maskensystem) wird damit auch mit Hubförderern möglich.

Das Verfahren ermöglicht die Anwendung einer einfachen und erprobten Technik bei gleichzeitiger Vermeidung von hohen Drehzahlen. Insbesondere ist daran gedacht, eine voneinander unabhängige Ansteuerung der Hubförderer zu realisieren.

Durch die phasenversetzte Ansteuerung der mindestens zwei Hubförderer kann die bei einem Betrieb von nur einem Hubförderer auftretende Veränderung des erzeugten Druckes in Abhängigkeit von der Durchführung einer Hubbewegung oder einer Rückhubbewegung sehr weitgehend kompensiert werden. Bereits bei einem Betrieb von nur zwei relativ zueinander phasenversetzt arbeitenden Hubförderern kann bei geeigneter Ansteuerung ein näherungsweise konstanter Druckverlauf generiert werden.

### Weiter auf Seite 4 der Ursprungsunterlagen

Gemäß einem typischen Verfahrensablauf ist vorgesehen, daß als Hubförderer Bälge verwendet werden.

Ebenfalls ist daran gedacht, daß als Hubförderer Kolben-zylinder-Anordnungen verwendet werden.

Zu einer sehr gleichmäßigen Druckerzeugung trägt es bei, daß der zweite Hubförderer einen Volumenfluß an Atemgas während einer Abbremsbewegung des ersten Hubförderers erzeugt.

Eine optimale Bewegungskoordinierung wird dadurch unterstützt, daß die Hubförderer von einer gemeinsamen Steuerung positioniert werden.

Eine erhöhte Genauigkeit bei der Bewegungskoordinierung kann dadurch erreicht werden, daß die Hubförderer von einer gemeinsamen Regelung positioniert werden.

Eine weitere Genauigkeitserhöhung kann dadurch erreicht werden, daß ein Innendruck der Hubförderer meßtechnisch erfaßt wird.

Eine Druckerfassung bei vermindertem gerätetechnischen Aufwand wird dadurch unterstützt, daß ein Differenzdruck zwischen den Hubförderern meßtechnisch erfaßt wird.

Die exakte Einhaltung von vorgegebenen Bewegungsabläufen wird dadurch unterstützt, daß eine jeweilige Positionierung der Hubförderer meßtechnisch erfaßt wird.

Zur Verringerung des gerätetechnischen Aufwandes wird vorgeschlagen, daß eine jeweilige Positionierung der Hubförderer unter Berücksichtigung einer Ansteuerung der Antriebe errechnet wird.

Ein modularer Geräteaufbau unter Verwendung von gleichartigen Funktionskomponenten wird dadurch unterstützt, daß von den Hubförderern im Wesentlichen gleich große Volumina gefördert werden.

Die Vorteile großvolumiger Hubförderer können mit den Vorteilen einer qualitativ hochwertigen Druckregelung dadurch kombiniert werden, daß vom ersten Hubförderer ein größeres Volumina als vom zweiten Hubförderer gefördert wird.

Eine exakte Drucksteuerung wird dadurch unterstützt, daß für die Hubförderer eine Ventilsteuerung verwendet wird.

Insbesondere ist daran gedacht, daß die Hubförderer mit einem Einlaßventil versehen sind.

Die Abgabe von Atemgas in Richtung an die Beatmungsmaske wird dadurch unterstützt, daß die Hubförderer mit einem Auslaßventil versehen sind.

Eine leicht steuerbare bzw. regelbare Ausführungsform wird dadurch bereitgestellt, daß der Antrieb als ein Elektromotor ausgebildet ist. Ebenfalls ist daran gedacht, daß der Antrieb pneumatisch oder hydraulisch ausgebildet ist.

In den Zeichnungen sind Ausführungsbeispiele der Erfindung schematisch dargestellt. Es zeigen:
- Fig. 1: Eine perspektivische Darstellung einer Beatmungseinrichtung mit Beatmungsmaske,
- Fig. 2: eine Prinzipdarstellung einer Vorrichtung zur Bereitstellung von Atemluft, die zwei Bälge mit jeweils zugeordneten Antrieben aufweist,
- Fig. 3: ein schematisches Blockschaltbild zur Veranschaulichung der wesentlichen Funktionskomponenten bei einer phasenversetzten Regelung der Einrichtung gemäß Fig. 2 und
- Fig. 4: ein Ablaufdiagramm zur Veranschaulichung der wesentlichen Prozeßabläufe bei einem Prozeßablauf innerhalb einer Einrichtung gemäß Fig. 3.

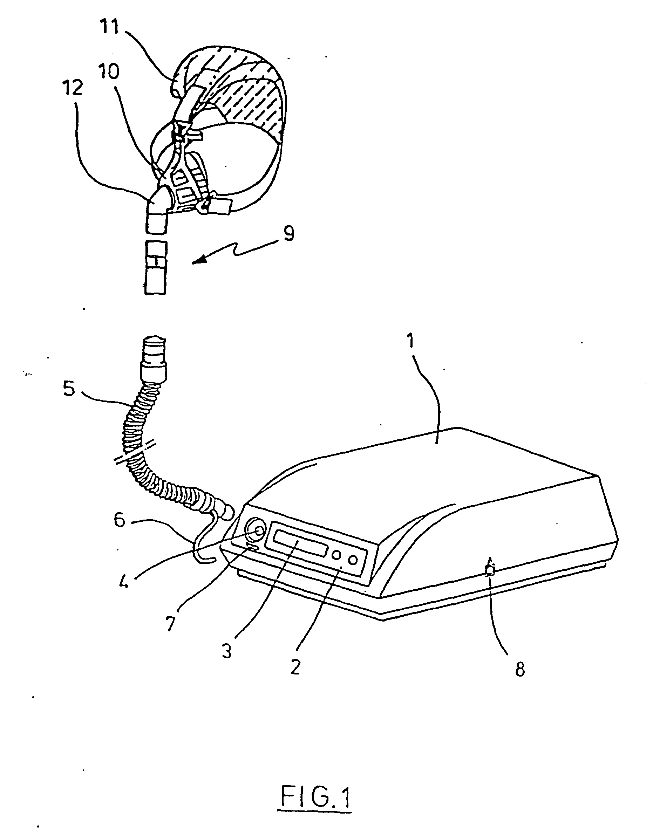

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Einrichtung zur Bereitstellung von Atemgas angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

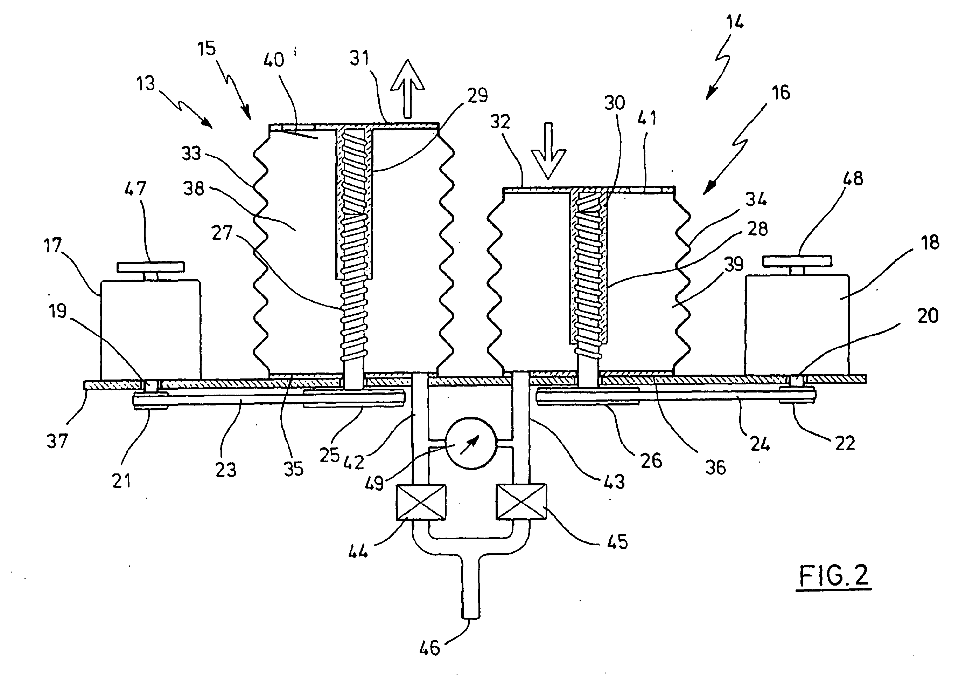

Fig. 2 zeigt eine schematische Darstellung einer Vorrichtung zur Bereitstellung von Atemgas, die mit zwei Hubförderern (13, 14) versehen ist. Die Hubförderer (13, 14) bestehen im Wesentlichen aus Bälgen (15, 16) sowie Antrieben (17, 18). Die Antriebe (17, 18) sind typischerweise als Elektromotoren ausgebildet. Auf Wellen (19, 20) der Antriebe (17, 18) aufsitzende Antriebsräder (21, 22) sind über Kopplungselemente (23, 24) mit Positionierrädern (25, 26) der Bälge (15, 16) verbunden. Die Positionierräder (25, 26) werden von Gewindestangen (27, 28) getragen, die in Gewindebuchsen (29, 30) der Bälge (15, 16) eingreifen.

Die Gewindebuchsen (29, 30) sind an Begrenzungsplatten (31, 32) der Bälge (15, 16) befestigt. Die Begrenzungsplatten (31, 32) sind über Balgelemente (33, 34) aus einem elastischen Material jeweils mit Grundplatten (35, 36) verbunden, die gemeinsam mit den Antrieben (17, 18) von einem Basiselement (37) gehaltert werden.

Die Innenräume (38, 39) der Bälge (15, 16) sind über Einlaßventile (40, 41) mit einer Umgebung verbunden. Die Einlaßventile (40, 41) können als Ventilklappen ausgebildet sein, die sich bei einer Ausfahrbewegung der Bälge (15, 16) automatisch öffnen und bei einer Kompression automatisch geschlossen werden. Alternativ ist auch eine Realisierung der Einlaßventile (40, 41) als elektromechanische Ventile möglich. Die Innenräume (38, 39) sind darüber hinaus über Auslaßleitungen (42, 43) und Auslaßventile (44, 45) mit einer gemeinsamen Atemgasleitung (46) verbunden.

Eine Drehbewegung der Antriebe (17, 18) wird über die Kopplungselemente (23, 24), die beispielsweise als Zahnriemen ausgebildet sein können, in eine Drehbewegung der Gewindestangen (27, 28) übertragen. Durch das Eingreifen der Gewindestangen (27, 28) in die Gewindebuchsen (29, 30) wird eine Rotation der Gewindestangen (27, 28) in eine Hubbewegung der Begrenzungsplatten (31, 32) transformiert.

Eine indirekte Positionserfassung der Begrenzungsplatten (31, 32) kann über Positionssensoren (47, 48) im Bereich der Antriebe (17, 18) erfolgen. Aufgrund der vorliegenden mechanischen Kopplung kann hierdurch ohne aufwendige Meßtechnik im Bereich der Bälge (15, 16) selbst eine ausreichend genaue Positionsbestimmung durchgeführt werden. Die Erfassung eines Differenzdruckes zwischen den Innenräumen (38, 39) der Bälge (15, 16) kann über einen Drucksensor (49) durchgeführt werden, der an die Auslaßleitungen (42, 43) angeschlossen ist. Es ist aber auch eine unmittelbare Druckmessung im Bereich der Innenräume (38, 39) möglich.

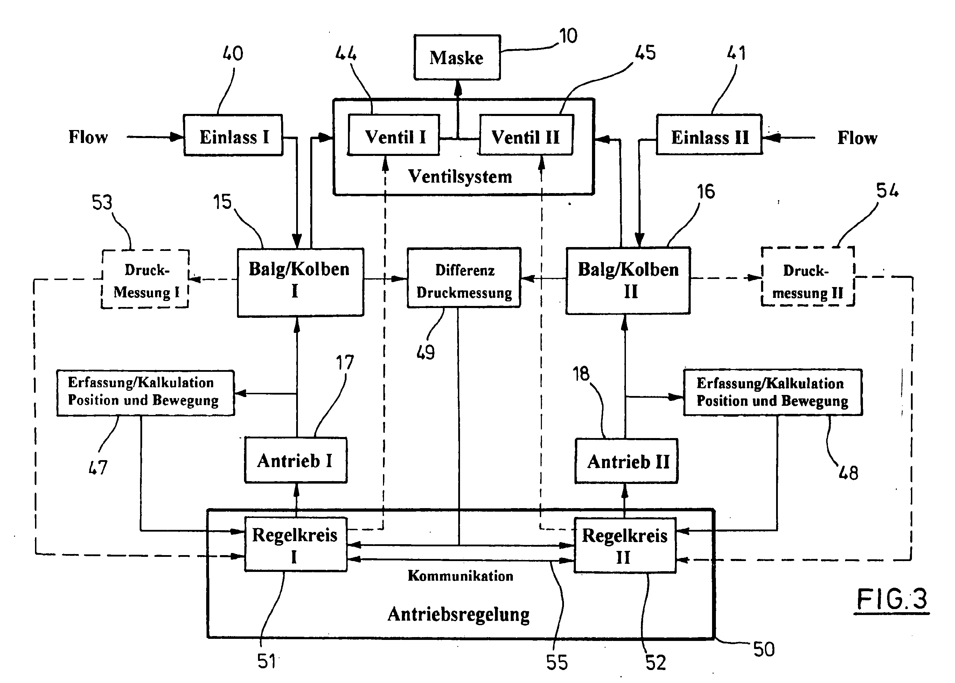

Fig. 3 veranschaulicht die grundsätzliche Ansteuerung der Anordnung gemäß Fig. 2. Es ist zu erkennen, daß die Antriebe (17, 18) an eine Bewegungskoordinierung (50) angeschlossen sind, die mit Regelkreisen (51, 52) ausgestattet ist. Den Regelkreisen (51, 52) wird als ein Istwert das Ausgangssignal des Drucksensors (49) zur Differenzdruckmessung zugeführt. Darüber hinaus können in den Regelkreisen (51, 52) Meßinformationen der Positionssensoren (47, 48) ausgewertet sowie von alternativen Drucksensoren (53, 54) zur Erfassung eines Druckes im Bereich der Innenräume (38, 39) der Bälge (15, 16) bereitgestellte Meßwerte berücksichtigt werden.

Zwischen den Regelkreisen (51, 52) kann eine Kommunikationsverbindung (55) bereitgestellt werden und es ist ebenfalls daran gedacht, von den Regelkreisen (51, 52) eine Steuerung der Ventile (44, 45) durchführen zu lassen.

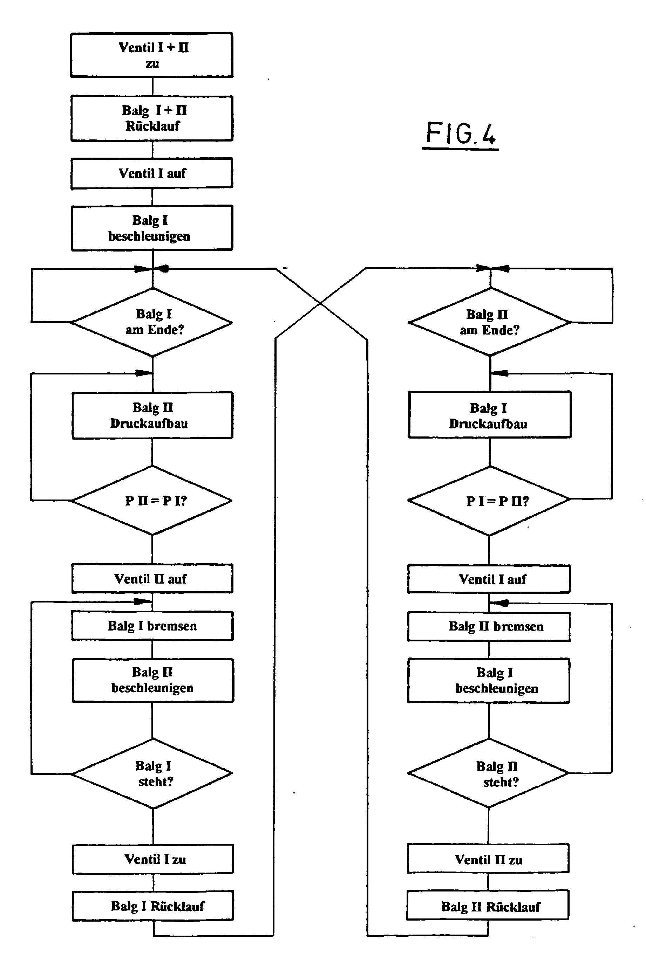

Das Ablaufdiagramm gemäß Fig. 4 veranschaulicht einen typischen Ablauf bei der Durchführung einer Regelung der Antriebe (17, 18) für die Hubförderer (13, 14). Es wird zunächst eine Synchronisationsphase durchlaufen, um unabhängig von einer jeweiligen Anfangspositionierung der Antriebe (17, 18) einen exakt koordinierten Verfahrensablauf zu gewährleisten. Zunächst werden beide Auslaßventile (44, 45) geschlossen und beide Bälge (15, 16) werden durch eine entsprechende Rotation der Gewindestangen (27, 28) mit ihren Begrenzungsplatten (31, 32) in eine ausgefahrene Positionierung überführt, so daß ein maximales Volumen der Innenräume (38, 39) bereitgestellt wird. Anschließend wird zunächst das Auslaßventil (44) des Balges (15) geöffnet und es erfolgt durch eine Aktivierung des Antriebes (17) ein Zurückziehen der Begrenzungsplatte (31) und damit eine Kontraktion des Balges (15). Nach Durchlauf dieser Verfahrensschritte ist die Initialisierungsphase mit der beginnenden Kontraktion des Balges (15) abgeschlossen.

Als Teil des kontinuierlich zu durchlaufenden Steuerungsvorganges wird dann über den Positionssensor (47) abgefragt, ob sich die Begrenzungsplatte (31) bis zu einem vorgegebenen Abstand an ihre maximal abgesenkte Endpositionierung angenähert hat. Solange dies noch nicht der Fall ist, wird die Kontraktionsbewegung des Balges (15) fortgesetzt. Nach Erreichen des vorgegebenen Abstandes zur maximalen Einfahrpositionierung erfolgt ein Beginn der Kontraktionsbewegung des Balges (16) durch Absenkung der zugehörigen Begrenzungsplatte (32). Hierdurch wird im Innenraum (39) des Balges (16) ein Druck aufgebaut.

Der Druckaufbau im Bereich des Balges (16) wird fortgesetzt, bis der Druck im Innenraum (39) den aktuellen Druck im Bereich des Innenraumes (38) erreicht hat. Dies kann entweder durch die Differenzdruckmessung mit Hilfe des Drucksensors (49) oder über die Drucksensoren (53, 54) meßtechnisch erfaßt werden. Solange im Bereich des Innenraumes (39) noch nicht der Druck wie im Bereich des Innenraumes (38) erreicht ist, wird die Kontraktionsbewegung des Balges (16) bei geschlossenem Auslaßventil (45) fortgesetzt. Nach Erreichen einer Druckgleichheit öffnet das Auslaßventil (45) und beide Bälge (15, 16) sind über ihre Auslaßventile (44, 45) an die Atemgasleitung (46) angeschlossen.

Nach Öffnung des Auslaßventils (45) wird die Kontraktionsbewegung des Balges (15) abgebremst und die Kontraktionsbewegung des Balges (16) wird beschleunigt. Dies erfolgt durch eine entsprechende Ansteuerung der Antriebe (17, 18). Dieser Vorgang wird fortgesetzt, bis die Begrenzungsplatte (31) des Balges (15) vollständig zur Ruhe gekommen ist. Bei Erreichen dieses Ruhezustandes wird das Auslaßventil (44) geschlossen und der Balg (15) wird durch eine entsprechende Ansteuerung des Motors (17) in eine Rückhubbewegung versetzt. Dies erfolgt durch ein erneutes Anheben der Begrenzungsplatte (31). Bei dieser Hubbewegung öffnet das Einlaßventil (40) und es strömt erneut Umgebungsluft in den Innenraum (38) des Balges (15).

Der Balg (16) hat nach dem Schließen des Auslaßventils (44) vollständig die Atemgasversorgung der Atemgasleitung (46) übernommen. Es erfolgt nunmehr im Hinblick auf den Balg (16) eine Absenkung der Begrenzungsplatte (32), bis diese sich bis zu einem vorgegebenen Abstand an ihre maximal abgesenkte Endpositionierung angenähert hat. Der oben für den Balg (15) beschriebene Ablauf wird nunmehr für den Balg (16) wiederholt. Nach einem Schließen des Auslaßventils (45) übernimmt wieder der Balg (15) die Versorgung der Atemgasleitung (46) mit Atemgas und der Füllvorgang für den Balg (16) wird eingeleitet. Anschließend werden die beschriebenen Zyklen jeweils abwechselnd durchlaufen.

Durch die verwendeten Sensoren kann über eine geeignete Ansteuerung bzw. Regelung der Antriebe (17, 18) sowie der Auslaßventile (44, 45) ein vorgegebenes Profil sowohl für den Druckverlauf als auch für den volumenfluß im Bereich der Atemgasleitung (46) exakt vorgegeben und eingehalten werden.

## Patentansprüche

1. Verfahren zu Bereitstellung von Atemgas, bei dem das Atemgas aus einer Umgebung angesaugt und von mindestens zwei Hubförderern (13, 14) in Richtung auf eine Beatmungsmaske (10) abgegeben wird, sowie bei dem die Hubförderer (13, 14) hinsichtlich ihrer Bewegungsabläufe relativ zueinander zeitlich phasenversetzt angesteuert werden und bei dem von einem zweiten Hubförderer (14) ein Druck vor einer Rücklaufbewegung eines ersten Hubförderers (13) erzeugt wird, **dadurch gekennzeichnet, daß** die Hubförderer (13, 14) von einer gemeinsamen Regelung positioniert werden und daß der erste Hubförderer (13) mit einem ersten Antrieb (17) und der zweite Hubförderer (14) mit einem zweiten Antrieb (18) gekoppelt wird.

2. Verfahren nach Anspruch 1, **dadurch gekennzeichnet, daß** als Hubförderer (13, 14) Bälge (15, 16) verwendet werden.

3. Verfahren nach Anspruch 1, **dadurch gekennzeichnet, daß** als Hubförderer (13, 14) Kolben-Zylinder-Anordnungen verwendet werden.

4. Verfahren nach einem der Ansprüche 1 bis 3, **dadurch gekennzeichnet, daß** der zweite Hubförderer (14) einen Volumenfluß an Atemgas während einer Abbremsbewegung des ersten Hubförderers (13) erzeugt.

5. Verfahren nach einem der Ansprüche 1 bis 4, **dadurch gekennzeichnet, daß** ein Innendruck der Hubförderer (13, 14) meßtechnisch erfaßt wird.

6. Verfahren nach einem der Ansprüche 1 bis 5, **dadurch gekennzeichnet, daß** ein Differenzdruck zwischen den Hubförderern (13, 14) meßtechnisch erfaßt wird.

7. Verfahren nach einem der Ansprüche 1 bis 6, **dadurch gekennzeichnet, daß** eine jeweilige Positionierung der Hubförderer (13, 14) meßtechnisch erfaßt wird.

8. Verfahren nach einem der Ansprüche 1 bis 7, **dadurch gekennzeichnet, daß** eine jeweilige Positionierung der Hubförderer (13, 14) unter Berücksichtigung einer Ansteuerung der Antriebe (17, 18) errechnet wird.

9. Verfahren nach einem der Ansprüche 1 bis 8, **dadurch gekennzeichnet, daß** von den Hubförderern (13, 14) im Wesentlichen gleich große Volumina gefördert werden.

10. Verfahren nach einem der Ansprüche 1 bis 8, **dadurch gekennzeichnet, daß** vom ersten Hubförderer (13) ein größeres Volumina als vom zweiten Hubförderer (14) gefördert wird.

11. Verfahren nach einem der Ansprüche 1 bis 10, **dadurch gekennzeichnet, daß** für die Hubförderer (13, 14) eine Ventilsteuerung verwendet wird.

12. Vorrichtung zur Bereitstellung von Atemgas, die mindestens zwei Hubförderer (13, 14) aufweist, die über mindestens eine Atemgasleitung an eine Beatmungsmaske (10) anschließbar sind, und bei der ein erster Hubförderer (13) mit einem ersten Antrieb (17) und ein zweiter Hubförderer (14) mit einem zweiten Antrieb (18) gekoppelt ist sowie bei der beide Antriebe (17, 18) an eine Bewegungskoordinierung (50) angeschlossen sind sowie von den Hubförderern (13, 14) zu durchlaufende Bewegungszyklen relativ zueinander einen zeitlichen Phasenversatz aufweisen, **dadurch gekennzeichnet, daß** die Bewegungskoordinierung (50) als Regelung ausgebildet ist.

13. Vorrichtung nach Anspruch 12, **dadurch gekennzeichnet, daß** die Hubförderer (13, 14) als Bälge (15, 16) ausgebildet sind.

14. Vorrichtung nach Anspruch 12, **dadurch gekennzeichnet, daß** die Hubförderer (13, 14) als Kolben-Zylinder-Anordnungen ausgebildet sind.

15. Vorrichtung nach einem der Ansprüche 12 bis 14 , **dadurch gekennzeichnet, daß** jedem der Hubförderer (13, 14) ein Drucksensor (53, 54) zugeordnet ist.

16. Vorrichtung nach einem der Ansprüche 12 bis 15, **dadurch gekennzeichnet, daß** die Hubförderer (13, 14) an einen Drucksensor (49) zur meßtechnischen Erfassung eines Differenzdruckes angeschlossen sind.

17. Vorrichtung nach einem der Ansprüche 12 bis 16, **dadurch gekennzeichnet, daß** zur Erfassung einer Positionierung mindestens eines der Hubförderer (13, 14) mindestens ein Positionssensor (47, 48) an die Bewegungskoordinierung (50) angeschlossen ist.

18. Vorrichtung nach einem der Ansprüche 12 bis 17, **dadurch gekennzeichnet, daß** die Hubförderer (13, 14) mit einem Einlaßventil (40, 41) versehen sind.

19. Vorrichtung nach einem der Ansprüche 12 bis 17, **dadurch gekennzeichnet, daß** die Hubförderer (13, 14) mit einem Auslaßventil (44, 45) versehen sind.

20. Vorrichtung nach einem der Ansprüche 12 bis 19, **dadurch gekennzeichnet, daß** der Antrieb (17, 18) als ein Elektromotor ausgebildet ist.

## Claims

1. Method for preparing respiratory gas, in which the respiratory gas is removed by suction from the surroundings and is delivered by at least two stroke conveyors (13, 14) in the direction of a breathing mask (10), and in which, with respect to their movement sequences, the stroke conveyors (13, 14) are controlled in a temporally phase-displaced manner relative to each other, and in which, before a return movement of a first stroke conveyor (13), a pressure is generated by a second stroke conveyor (14), **characterised in that** the stroke conveyors (13, 14) are positioned by a common control, and **in that** the first stroke conveyor (13) is coupled to a first drive (17) and the second stroke conveyor (14) is coupled to a second drive (18).

2. Method according to claim 1, **characterised in that** bellows (15, 16) are used as the stroke conveyors (13, 14).

3. Method according to claim 1, **characterised in that** piston-cylinder arrangements are used as the stroke conveyors (13, 14).

4. Method according to any one of claims 1 to 3, **characterised in that** the second stroke conveyor (14) generates a volume flow of respiratory gas during a deceleration movement of the first stroke conveyor (13).

5. Method according to any one of claims 1 to 4, **characterised in that** an internal pressure of the stroke conveyors (13, 14) is measured metrologically.

6. Method according to any one of claims 1 to 5, **characterised in that** a differential pressure between the stroke conveyors (13, 14) is measured metrologically.

7. Method according to any one of claims 1 to 6, **characterised in that** a respective positioning of the stroke conveyors (13, 14) is measured metrologically.

8. Method according to any one of claims 1 to 7, **characterised in that** a respective positioning of the stroke conveyors (13, 14) is calculated by taking into account activation of the drives (17, 18).

9. Method according to any one of claims 1 to 8, **characterised in that** volumes that are substantially equal in size are conveyed by the stroke conveyors (13, 14).

10. Method according to any one of claims 1 to 8, **characterised in that** a larger volume is conveyed by the first stroke conveyor (13) than by the second stroke conveyor (14).

11. Method according to any one of claims 1 to 10, **characterised in that** a valve controller is used for the stroke conveyors (13, 14).

12. Device for preparing respiratory gas, comprising at least two stroke conveyors (13, 14) which can be connected by at least one respiratory gas line to a breathing mask (10), and in which a first stroke conveyor (13) is coupled to a first drive (17) and a second stroke conveyor (14) is coupled to a second drive (18), and in which two drives (17, 18) are connected to a movement coordination (50) and movement cycles that are to be passed through by the stroke conveyors (13, 14) have a temporal phase displacement relative to each other, **characterised in that** the movement coordination (50) is constructed as a control.

13. Device according to claim 12, **characterised in that** the stroke conveyors (13, 14) are constructed as bellows (15, 16).

14. Device according to claim 12, **characterised in that** the stroke conveyors (13, 14) are constructed as piston-cylinder arrangements.

15. Device according to any one of claims 12 to 14, **characterised in that** a pressure sensor (53, 54) is associated with each of the stroke conveyors (13, 14).

16. Device according to any one of claims 12 to 15, **characterised in that** the stroke conveyors (13, 14) are connected to a pressure sensor (49) for metrological measurement of a differential pressure.

17. Device according to any one of claims 12 to 16, **characterised in that** at least one position sensor (47, 48) is connected to the movement coordination (50) for detecting positioning of at least one of the stroke conveyors (13, 14).

18. Device according to any one of claims 12 to 17, **characterised in that** the stroke conveyors (13, 14) are provided with an intake valve (40, 41).

19. Device according to any one of claims 12 to 17, **characterised in that** the stroke conveyors (13, 14) are provided with an outlet valve (44, 45).

20. Device according to any one of claims 12 to 19, **characterised in that** the drive (17, 18) is constructed as an electric motor.

## Revendications

1. Procédé pour fournir du gaz respiratoire, dans lequel le gaz respiratoire est aspiré dans l'environnement et est délivré dans un masque respiratoire (10) par au moins deux dispositifs de refoulement (13, 14) qui travaillent en va-et-vient, dans lequel les déplacements des dispositifs de refoulement (13, 14) qui travaillent en va-et-vient sont commandés en déphasage mutuel, dans lequel la pression avant un déplacement de recul du premier dispositif de refoulement (13) qui travaille en va-et-vient est créée par le deuxième dispositif de refoulement (14) qui travaille en va-et-vient, **caractérisé en ce que** les dispositifs de refoulement (13, 14) qui travaillent en va-et-vient sont positionnés par une régulation commune et **en ce que** le premier dispositif de refoulement (13) qui travaille en va-et-vient est accouplé à un premier entraînement (17) et le deuxième dispositif (14) qui travaille en va-et-vient est accouplé à un deuxième entraînement (18).

2. Procédé selon la revendication 1, **caractérisé en ce que** comme dispositifs de refoulement (13, 14) qui travaillent en va-et-vient, on utilise des soufflets (15, 16).

3. Procédé selon la revendication 1, **caractérisé en ce que** comme dispositifs de refoulement (13, 14) qui travaillent en va-et-vient, on utilise des agencements à cylindre et piston.

4. Procédé selon l'une des revendications 1 à 3, **caractérisé en ce que** le deuxième dispositif de refoulement (14) qui travaille en va-et-vient crée un débit volumique d'air respiratoire pendant le déplacement de freinage du premier dispositif de refoulement (13) qui travaille en va-et-vient.

5. Procédé selon l'une des revendications 1 à 4, **caractérisé en ce que** la pression intérieure dans les dispositifs de refoulement (13, 14) qui travaillent en va-et-vient est déterminée par une technique de mesure.

6. Procédé selon l'une des revendications 1 à 5, **caractérisé en ce que** la différence de pression entre les dispositifs de refoulement (13, 14) qui travaillent en va-et-vient est déterminée par une technique de mesure.

7. Procédé selon l'une des revendications 1 à 6, **caractérisé en ce que** le positionnement des dispositifs de refoulement (13, 14) qui travaillent en va-et-vient est détecté à tout moment par une technique de mesure.

8. Procédé selon l'une des revendications 1 à 7, **caractérisé en ce que** le positionnement des dispositifs de refoulement (13, 14) qui travaillent en va-et-vient est calculé à tout moment en tenant compte de la commande des entraînements (17, 18).

9. Procédé selon l'une des revendications 1 à 8, **caractérisé en ce que** des débits essentiellement identiques sont refoulés par les dispositifs de refoulement (13, 14) lorsqu'ils travaillent en va-et-vient.

10. Procédé selon l'une des revendications 1 à 8, **caractérisé en ce que** le débit refoulé par le premier dispositif de refoulement (13) qui travaille en va-et-vient est plus élevé que celui refoulé par le deuxième dispositif de refoulement (14) qui travaille en va-et-vient.

11. Procédé selon l'une des revendications 1 à 10, **caractérisé en ce que** l'on utilise une commande par soupape pour les dispositifs de refoulement (13, 14) qui travaillent en va-et-vient.

12. Dispositif pour délivrer du gaz respiratoire, lequel dispositif présente au moins deux dispositifs de refoulement (13, 14) qui travaillent en va-et-vient et qui peuvent être raccordés à un masque respiratoire (10) par au moins un conduit de gaz respiratoire, et dans lequel un premier dispositif de refoulement (13) qui travaille en va-et-vient est accouplé à un premier entraînement (17) et un deuxième dispositif de refoulement (14) qui travaille en va-et-vient est accouplé à un deuxième entraînement (18), les deux entraînements (17, 18) étant raccordés à une coordination (50) du déplacement, les cycles de déplacement des dispositifs de refoulement (13, 14) qui travaillent en va-et-vient présentant un décalage de phase l'un par rapport à l'autre, **caractérisé en ce que** la coordination (50) du déplacement est configurée sous la forme d'une régulation.

13. Dispositif selon la revendication 12, **caractérisé en ce que** les dispositifs de refoulement (13, 14) qui travaillent en va-et-vient sont configurés comme soufflets (15, 16).

14. Dispositif selon la revendication 12, **caractérisé en ce que** les dispositifs de refoulement (13, 14) qui travaillent en va-et-vient sont configurés sous la forme d'agencements à piston et cylindre.

15. Dispositif selon l'une des revendications 12 à 14, **caractérisé en ce qu'**une sonde de pression (53, 54) respective est associée à chaque dispositif de refoulement (13, 14) qui travaille en va-et-vient.

16. Dispositif selon l'une des revendications 12 à 15, **caractérisé en ce que** les dispositifs de refoulement (13, 14) qui travaillent en va-et-vient sont raccordés à une sonde de pression (49) qui détecte la différence de pression par une technique de mesure.

17. Dispositif selon l'une des revendications 12 à 16, **caractérisé en ce que** pour détecter le positionnement d'au moins l'un des dispositifs de refoulement (13, 14) qui travaille en va-et-vient, au moins une sonde de position (47, 48) est raccordée à la coordination (50) du déplacement.

18. Dispositif selon l'une des revendications 12 à 17, **caractérisé en ce que** les dispositifs de refoulement (13, 14) qui travaillent en va-et-vient sont dotés d'une soupape d'admission (40, 41).

19. Dispositif selon l'une des revendications 12 à 17, **caractérisé en ce que** les dispositifs de refoulement (13, 14) qui travaillent en va-et-vient sont dotés d'une soupape de sortie (44, 45).

20. Dispositif selon l'une des revendications 12 à 19, **caractérisé en ce que** l'entraînement (17, 18) est configuré comme moteur électrique.
